# EUROPÄISCHE PATENTSCHRIFT

(11) **EP 1 435 339 B1**
(45) Veröffentlichungstag und Bekanntmachung des Hinweises auf die Patenterteilung: **07.03.2007**
(21) Anmeldenummer: 04008188.7
(22) Anmeldetag: 08.11.2002
(51) Int. Cl.: C02F 1/34

(54) **Verfahren und Vorrichtung zur Behandlung von Abwasser, Klärschlamm und organischen Substraten**
Method and device for the treatment of effluent, sludge and organic substrates
Procédé et dispositif de traitement de substrats organiques, de boues résiduaires et d'eaux usées

(30) Priorität: 12.11.2001 DE 10155161
(43) Veröffentlichungstag der Anmeldung: 07.07.2004
(62) Teilanmeldung aus: 02781155.3
(73) Patentinhaber: WILO AG, 44263 Dortmund (DE)
(72) Erfinder: Schmid, Andreas, Dr., 95213 Münchberg (DE); Geier, Wolfgang, 95030 Hof (DE)
(74) Vertreter: LOUIS, PÖHLAU, LOHRENTZ

(56) Entgegenhaltungen:
- DE-A- 4 114 694
- GB-A- 1 589 551
- US-A- 4 341 632
- US-A- 5 494 585
- SCHMELZ K-G, WINTER A: "Klärschlammverminderung durch verschiedene Methoden der Desintegration auf der Kläranlage Schermbeck" 18. BOCHUMER WORKSHOP SIEDLUNGSWASSERWIRTSCHAFT: INNOVATIONEN IN DER ABWASSERBESEITIGUNG, SCHRIFTENREIHE SIEDLUNGSWASSERWIRTSCHAFT BOCHUM, 21. September 2000 (2000-09-21), Seiten 205-224, XP002231503
- MÜLLER ET AL: "Verfahren und Anwendungsgebiete der mechanischen Klärschlammdesintegration" KA-WASSERWIRTSCHAFT, ABWASSER, ABFALL, Bd. 47, Nr. 4, 1. April 2000 (2000-04-01), Seiten 570-576, XP002231544

## Beschreibung

Die Vorrichtung betrifft ein Verfahren zur Behandlung von fädige und flockenbildende Mikroorganismen-haltigem Abwasser, Klärschlamm oder in Biogasanlagen verwendeten organischen Substraten zur Verringerung oder Vermeidung der Bildung von Blähschlamm, Schwimmschlamm und/oder Schaum.

Bei Kläranlagen mit biologischen Reinigungsstufen kommt es immer wieder zu Betriebsproblemen, die auf nicht sedimentierende bzw. flotierende Schlammaggregate insbesondere im Belebungsbecken und im Nachklärbecken zurückzuführen sind.

Für einen stabilen Betrieb einer Kläranlage ist es erforderlich, daß die zur Reinigung des Abwassers verwendete Biomasse, die die zur Reinigung verwendeten Mikroorganismen umfaßt, von dem gereinigten Abwasser getrennt wird. Bei dem Belebungsverfahren wird die Biomasse in der Regel in einem Nachklärbecken durch Sedimentation abgetrennt und teilweise oder vollständig zum Belebungsbecken zurückgeführt. Ein störungsfreier Betrieb einer Kläranlage mit biologischer Reinigungsstufe erfordert daher eine gut sedimentierende Biomasse bzw. einen gut sedimentierenden Belebtschlamm.

Die in dem Belebungsbecken verwendete Biomasse ist eine Mischbiozönose verschiedenster Mikroorganismen. In der Mischbiozönose sind unter anderem fädige Bakterien bzw. Fadenbakterien und flocken bildende Bakterien enthalten.

Zu einer Entstehung von nicht sedimentierenden oder aufschwimmenden Schlammaggregaten kommt es bei einem vermehrten Wachstum von fädigen Bakterien, die dann zu einem Fadengeflecht aggregieren. Dieses Fadengeflecht behindert den Eindick- und Absetzvorgang des belebten Schlamms erheblich. Die aufschwimmenden Schlammaggregate bestehen überwiegend aus Blähschlamm, Schwimmschlamm und Schaum.

Blähschlamm, Schwimmschlamm und Schaum können weiterhin entstehen, wenn in dem Abwasser fein verteilte Gasbläschen, hydrophobe Abwasserinhaltsstoffe und/oder Zellstrukturen sowie oberflächenaktive Substanzen enthalten sind.

Ein stabiler Betrieb einer biologischen Kläranlage ist bei einer Bildung von Blähschlamm, Schwimmschlamm oder Schaum sehr erschwert bzw. nicht möglich.

Aus der DE 198 43 862 A1 ist ein Verfahren zur Verhinderung der Schwimm- und/oder Blähschlammbildung in Kläranlagen bekannt, bei dem der biomassehaltige Schlamm mit Ultraschall behandelt wird.

Aus der DE 195 17 381 C1 ist eine weitere Einrichtung zum Zerstören zellulärer Strukturen in Schlämmen biologischer Kläranlagen unter Verwendung einer Ultraschallbeaufschlagung bekannt.

Nachteilig ist, daß diese Verfahren in technischer Hinsicht aufwendig sind und an das Bedienungspersonal erhöhte Anforderungen gestellt werden.

In KA-Wasserwirtschaft, Abwasser, Abfall 2001 (48) Nr. 5, Seiten 598 bis 604 wird der Einsatz von Füll- und Flockungsmitteln auf Poly-Aluminium-Basis, wie bspw. Poly-Aluminiumhydroxid oder Poly-Aluminiumchloride, diskutiert. Die Verwendung solcher Füll- und Flockungsmittel ist jedoch mit erheblichen Mehrkosten verbunden.

In WAP (Wasser Abwasser Praxis) 3/99 Seiten 25 bis 31 wird offenbart, daß durch mechanische Zerkleinerung von Bläh- und Schwimmschlämmen kleinere Aggregate erzeugt werden, die verbesserte Sedimentationseigenschaften aufweisen.

Die Zerkleinerung des Bläh- und Schwimmschlammes erfolgt unter Verwendung einer Rührwerkskugelmühle, einem Scherspalt-, einem Ultraschall- und einem Hochdruckhomogenisator. Um eine wirksame Behandlung des Schwimmschlamms durchführen zu können, ist ein Energieeintrag von wenigstens etwa 1000 kJ/m³ Schlamm erforderlich.

Nachteilig ist, daß die in dieser Veröffentlichung vorgeschlagenen Zerkleinerungsgeräte aufwendig und kostenintensiv sind und sich daher insbesondere für viele kleine kommunale Kläranlagen nicht eignen.

Aus SCHMELZ K.-G., WINTER A., "Klärschlammverminderung durch verschiedene Methoden der Desintegration auf der Kläranlage Schermbeck" 18. Bochumer Workshop Siedlungswasserwirtschaft: Innovationen in der Abwasserbeseitigung, Schriftenreihe Siedlungswasserwirtschaft Bochum, 21. September 2000, Seiten 205-224 sind Hochdruckhomogenisatoren bekannt, bei denen unter Verwendung von Kavitation Zellwände zerstört werden.

Aufgabe der Erfindung ist es, ein Verfahren und eine Vorrichtung bereitzustellen, das bzw. die einfach in der Handhabung und preiswert im Betrieb ist sowie wirksam die Bildung von nicht sedimentierenden Schlammaggregaten in Kläranlagen, Biogasreaktoren etc. verringert oder verhindert.

Die der Erfindung zugrundeliegende Aufgabe wird durch ein Verfahren zur Behandlung von fädige und flockenbildende Mikroorganismen-haltigem Abwasser, Klärschlamm oder in Biogasanlagen verwendeten organischen Substraten gemäß Anspruch 1 gelöst.

Bevorzugte Weiterbildungen sind in den Unteransprüchen 2 bis 13 angegeben.

Nachstehend wird die Erfindung im wesentlichen unter Bezug auf Kläranlagen beschrieben. Die Erfindung ist jedoch keinesfalls auf Kläranlagen beschränkt. Vielmehr kann die Erfindung in weiteren biologischen Anlagen verwendet werden, in denen fädige und flockenbildende Mikroorganismen in einer Mischbiozönose vorliegen und zusammenwirken. Die Erfindung ist beispielsweise auch bei Biogasreaktoren und Anaerobfermentern verwendbar. Insofern gelten die nachstehenden Ausführungen entsprechend für Biogasreaktoren und Anaerobfermenter.

Das Abwasser ist im Sinne der Erfindung bevorzugt ein Belebtschlamm oder eine Mischbiozönose in biologischen Kläranlagen.

Gemäß der vorliegenden Erfindung kann das seit Jahrzehnten bestehende Problem der Entstehung von Blähschlamm, Schwimmschlamm und Schaum in biologischen Reinigungsstufen von Kläranlagen auf überraschend einfache Art und Weise gelöst werden.

Gemäß der vorliegenden Erfindung wird das Abwasser, bevorzugt Belebtschlamm oder eine Mischbiozönose, in einem Strömungskanal einer Scherbeanspruchung durch hydrodynamisch erzeugte Scherfelder unterworfen. Die Scherfelder entstehen durch im im Strömungskanal geführten Abwasser, bevorzugt Belebtschlamm oder eine Mischbiozönose, auftretende Geschwindigkeitsgradienten.

Die Scherbeanspruchung führt zu einer Zerkleinerung bzw. Zerteilung von aus langfädigen Mikroorganismen gebildeten Schlammaggregaten oder Fadengeflechten. Weiterhin werden in diesen Schlammaggregaten eingeschlossene oder anhaftende fein verteilte Gasblasen freigesetzt. Diese in den unbehandelten Schlammaggregaten enthaltenen Gasblasen tragen zu einer Verschlechterung des Sedimentationsverhaltens der Schlammaggregate bei.

Schließlich kommt es durch die Scherbeanspruchung zu einer teilweisen Zerstörung der fädigen Bakterien und zu einer Verkürzung der Filamentlänge der fädigen Bakterien.

Der auf die in der Mischbiozönose enthaltenen fädigen Bakterien durch das erzeugte Scherfeld ausgeübte Streß führt insbesondere zu Veränderungen im Metabolismus der fädigen Bakterien, was zu einer Veränderung der Wachstumskinetik bei den fädigen Mikroorganismen bzw. Bakterien führt. Das Wachstum der fädigen Mikroorganismen wird deutlich verringert.

Die Veränderung im Metabolismus von fädigen Mikroorganismen ist insbesondere auf die Stimulierung von Schutzmechanismen gegenüber dem durch die angelegte Scherbeanspruchung erzeugten Streß zurückzuführen. Dies führt u.a. zu einer Sekretion von extrazellulären polymeren Substanzen, die auch als EPS bezeichnet werden, vorwiegend durch die fädigen Mikroorganismen. Die sekretierten extrazellulären polymeren Substanzen bewirken vorteilhaft eine Adhäsion der im Scherfeld zerkleinerten Schlammaggregate. Diese Adhäsion wirkt der Entstehung zu kleiner Schlammpartikel entgegen, die nicht oder zu langsam sedimentieren, und führt zu einer vorteilhaften Flockung und mithin zu der gewünschten Sedimentation der Mischbiozönose.

Die relativen Anteile von flockenbildenden Mikroorganismen zu fädigen Mikroorganismen in der Mischbiozönose bzw. dem Klärschlamm ändern sich zugunsten der flockenbildenden Mikroorganismen. Ein Erhöhung des Verhältnisses von flockenbildenden Bakterien zu fädigen Bakterien in der Mischbiozönose begünstigt eine Sedimentation der Schlammaggregate.

Flockenbildende Mikroorganismen zeigen im Unterschied zu fädigen Mikroorganismen ein gutes Sedimentationsverhalten.

Die Scherbeanspruchung wird durch eine turbulente Strömung in dem Strömungskanal erzeugt. Erfindungsgemäß ist in dem Strömungskanal wenigstens eine Verengung angeordnet, durch die das Abwasser geführt wird.

Das in dem Strömungskanal geführte Abwasser ist hochturbulent. Die Reynoldszahl des turbulenten Abwassers bzw. der Mischbiozönose beträgt bevorzugt wenigsten 100.000, weiter bevorzugt wenigstens 180.000, noch weiter bevorzugt wenigstens 250.000, noch bevorzugter wenigstens 500.000.

Das Abwasser, bevorzugt Belebtschlamm oder eine Mischbiozönose, kann mit Hilfe einer herkömmlichen Pumpe durch den Strömungskanal gepumpt werden. Die wenigstens eine Verengung kann dabei sowohl druck- als auch saugseitig im Strömungskanal angeordnet sein.

Erfindungsgemäß wird die Fließgeschwindigkeit des Abwassers bei Durchtritt durch die Verengung im Strömungskanal unter abnehmenden Druck erhöht, bis Dampfdruck erreicht wird, was sich in Form von Kavitation ausbildet.

Als Kavitation wird die Entstehung von Hohlräumen in Flüssigkeiten und deren Bewegung bezeichnet. Der Hohlraum kann durch eine lokale Unterdruckphase, Scherspannungen oder einen Energieeintrag in die Flüssigkeit gerissen werden. In die so entstandenen Blasen diffundieren die in der Flüssigkeit gelösten Gase hinein. Wenn der Druck in der Flüssigkeit wieder ansteigt, kollabieren die Gasblasen durch den äußeren Druck. Während des Blasenkollaps wird der Blaseninhalt so stark komprimiert, daß hohe Drücke, bis zu einigen 1000 bar, und hohe Temperaturen, bis zu mehreren 1000 K, lokal erzeugt werden. Das Gas wird in der Blase ionisiert und Radikale entstehen.

Des weiteren emittiert die Blase während des Kollaps Schockwellen und, sofern sie sich in der Nähe von festen Oberflächen befindet, einen Flüssigkeitsjet, der durch die Blase hindurchtritt. Die von den Blasen emittierten Schockwellen und Flüssigkeitsjets haben die Kraft, um Partikelagglomerate, wie beispielsweise Schlammaggregate, aufzulösen sowie Makromoleküle und bakterielle Zellwände aufzubrechen.

In der in dem Strömungskanal angeordneten Verengung nimmt die Fließgeschwindigkeit zu und der Druck ab. Unter diesen Bedingungen entsteht die bei dem erfindungsgemäßen Verfahren erwünschte Kavitation.

Bei dem erfindungsgemäßen Verfahren kommt es zu einem gemeinsamen Auftreten von Scherbeanspruchung und Kavitation.

Die bspw. durch eine hochturbulente Strömung erzeugte Scherbeanspruchung sowie die durch die Verengung in dem Strömungskanal erzeugte Kavitation wirken bei dem erfindungsgemäßen Verfahren äußerst vorteilhaft zusammen.

Die Scherbeanspruchung und die Kavitation wirken sich insbesondere auf die größeren fädigen Mikroorganismen bzw. auf die von fädigen Mikroorganismen gebildeten Fadengeflechte aus. Die fädigen Mikroorganismen werden teilweise durch Aufbrechen der Zellmembranen zerstört. Zu einem wesentlichen Teil kommt es zu einer Verkürzung der Filamente und somit zu einem Aufbrechen bzw. einer Zerkleinerung der Fadengeflechte.

Überraschenderweise werden die flockenbildenden Bakterien durch das erfindungsgemäße Verfahren nicht zerstört. Ferner kommt es zu keiner wesentlichen Änderung im Zellmetabolismus, so daß die Wachstumskinetik der flockenbildenden Mikroorganismen nicht wesentlich beeinträchtigt bzw. nicht wesentlich verändert wird. Somit führt das erfindungsgemäße Verfahren im Hinblick auf die unterschiedlichen Wachstumskinetiken von fädigen und flockenbildenden Mikroorganismen zu einer vorteilhaften Veränderung der Zusammensetzung der Mischbiozönose. Diese durch das erfindungsgemäße Verfahren bewirkte Veränderung der bakteriellen Zusammensetzung führt mithin zu einer Gesundung des Klärschlamms.

Gemäß einer weiteren Ausführungsform der Erfindung ist die Dauer der Kavitation und damit die Einwirkdauer der Kavitation auf das Abwasser, bzw. die Mischbiozönose einstellbar.

Es wurde überraschenderweise herausgefunden, daß die durch die Verengung des Strömungskanals hervorgerufene Kavitation erhalten werden kann, wenn der Innendurchmesser des Strömungskanals, der in bezug auf die Fließrichtung des Abwassers, nach der Verengung angeordnet ist, größer ist als der kleinste Innendurchmesser der Verengung in dem Strömungskanal, aber kleiner ist als der Innendurchmesser des Strömungskanals, der vor der Verengung angeordnet ist.

Über die Länge des nach der Verengung angeordneten Strömungskanals, kann die Dauer der Kavitation gesteuert werden. Wenn der nach der Verengung angeordnete Strömungskanal verlängert wird, verlängert sich auch die Einwirkdauer auf das Abwasser, bevorzugt den Belebtschlamm oder die Mischbiozönose.

Das Verhältnis des Innendurchmessers des Strömungskanals vor der Verengung, bezogen auf die Fließrichtung des Abwassers, zu dem Innendurchmesser des Strömungskanals nach der Verengung kann bspw. etwa 5:1 bis etwa 1,2:1, bevorzugter 3:1 bis 1,5:1, sehr bevorzugt 2:1, betragen.

Das Verhältnis des kleinsten Innendurchmessers der Verengung zu dem Innendurchmesser des in bezug auf die Verengung abströmseitig angeordneten Strömungskanals kann beispielsweise 1:1,2 bis 1:3, bevorzugt 1:2, betragen.

Äußerst überraschend ist, daß durch die erzeugte Kavitation bei dem erfindungsgemäßen Verfahren weder an der Verengung im Strömungskanal noch an dem in Fließrichtung des Abwassers nachgeordneten Strömungskanal mit gegebenenfalls angeordneten Armaturen Abrasion verursacht wird.

Es wird vermutet, daß die Kavitationsblasen aufgrund eines höheren Drucks an der Wandung des Strömungskanals überwiegend in der Mitte des Strömungskanals auftritt. Die in der Mitte der Abwasserströmung auftretenden Kavitationsblasen kommen mithin nicht in Kontakt mit der Wandung des Strömungskanals und können insoweit zu keiner Abrasion führen.

Im einfachsten Fall kann der Strömungskanal als Rohrleitung ausgebildet sein. Die in dem Strömungskanal vorgesehene Verengung kann im einfachsten Fall als Düse, vorzugsweise in der Geometrie einer Lavaldüse, ausgebildet sein.

Die der Erfindung zugrundeliegende Aufgabe wird weiterhin durch eine Vorrichtung zur Behandlung von fädige und flockenbildende Mikroorganismen-haltigem Abwasser, Klärschlamm oder in Biogasanlagen verwendeten organischen Substraten gemäß Anspruch 14 gelöst.

Bevorzugte Weiterbildungen der Vorrichtungen sind in den Unteransprüchen 15 bis 25 angegeben.

In bezug auf die Größenverhältnisse der Innendurchmesser des Strömungskanals an der Verengung des Strömungskanals bzw. vor und nach der Verengung wird auf die obigen Ausführungen zu dem erfindungsgemäßen Verfahren verwiesen.

Erfindungsgemäß umfaßt die wenigstens eine Verengung einen Abschnitt mit konvergierendem und divergierendem Innendurchmesser, wobei, bezogen auf die Fließrichtung des Abwassers, bevorzugt Belebtschlamm oder ein Mischbiozönose, der Abschnitt mit konvergierendem Innendurchmesser vor dem Abschnitt mit divergierendem Innendurchmesser angeordnet ist.

Der Strömungskanal kann sich bspw., bezogen auf die Fließrichtung des Abwassers, zunächst konisch verengen bis zu einem geringsten Innendurchmesser und nachfolgend konisch erweitern. Die Verengung ist bevorzugt eine Düse, die zwei Strömungskanäle, bevorzugt Rohrleitungen, miteinander verbindet.

Bei einer weiteren Ausführungsform der Erfindung ist die Verengung oder die Düse so ausgebildet, daß die Länge des Abschnitts mit konvergierendem Innendurchmesser kürzer ist als die Länge des Abschnitts mit divergierendem Innendurchmesser.

Bei dieser Ausführungsform ist die geometrische Ausgestaltung der Verengung oder der Düse mithin nicht symmetrisch zu dem Abschnitt mit dem geringsten Innendurchmesser der Verengung oder der Düse.

Es hat sich gezeigt, daß bei asymmetrischer Ausgestaltung der Düse die gewünschte Kavitation so eingestellt werden kann, daß eine für den Zweck der Vermeidung von Blähschlamm, Schwimmschlamm und/oder Schaum geeignete Kavitation erzeugt wird. Insbesondere läßt sich über den Austrittswinkel der Düse die Länge der nachfolgenden Kavitationsbildung und mithin die Einwirkdauer der Kavitation auf das Abwasser steuern.

Gemäß einer bevorzugten Ausführungsform weist die Düse die Geometrie einer Lavaldüse auf.

Bei einer weiteren Ausführungsform der erfindungsgemäßen Vorrichtung ist abströmseitig von der Verengung oder der Düse in dem Strömungskanal eine Drossel, beispielsweise ein Schieber, angeordnet. Über diese Drossel kann das Auftreten von Kavitation zusätzlich gesteuert werden.

In den Strömungskanal eintretende Gasblasen können einen dämpfenden Effekt auf die Kavitationsbildung ausüben. Insofern wird gemäß einer bevorzugten Ausführungsform der Anteil von Gasblasen im Abwasser, das in den Strömungskanal eingebracht wird, verringert. Bevorzugt wird das Abwasser während des Förderns in den Strömungskanal entgast, beispielsweise durch Verwendung einer Pumpe, bevorzugt einer Tauchmotorpumpe, in einem getauchten Schacht.

Die Erfindung wird nachfolgend anhand der Figuren 1 bis 8 weiter veranschaulicht. Dabei zeigt
Figur 1 eine schematische Darstellung eines beispielhaften Aufbaus einer erfindungsgemäßen Vorrichtung,
Figur 2 eine schematische Darstellung der Entgasung von Schlammaggregaten im Scherfeld,
Figur 3 eine schematische Darstellung einer durch Sekretion von extrazellulären polymeren Substanzen (EPS) bewirkten verbesserten Flockung von zerkleinerten Schlammaggregaten,
Figur 4 eine schematische Darstellung einer Verkürzung der Filamente von fädigen Mikroorganismen im Scherfeld und gegebenenfalls durch Kavitation,
Figur 5 eine schematische Darstellung des Einflusses des erfindungsgemäßen Verfahrens auf die Wachstumskinetik von flockenbildenden Mikroorganismen und fädigen Mikroorganismen,
Figur 6 eine Darstellung von Meßwerten des Schlammvolumenindexes (ISV) vor und nach Anwendung des erfindungsgemäßen Verfahrens in einer Versuchskläranlage,
Figur 7 eine Darstellung von Meßwerten, die eine Stabilisierung der Prozeßparameter durch das erfindungsgemäße Verfahren in einer Versuchskläranlage zeigen, und
Figur 8 eine schematische Darstellung einer in einem getauchten Schacht angeordneten Pumpe.

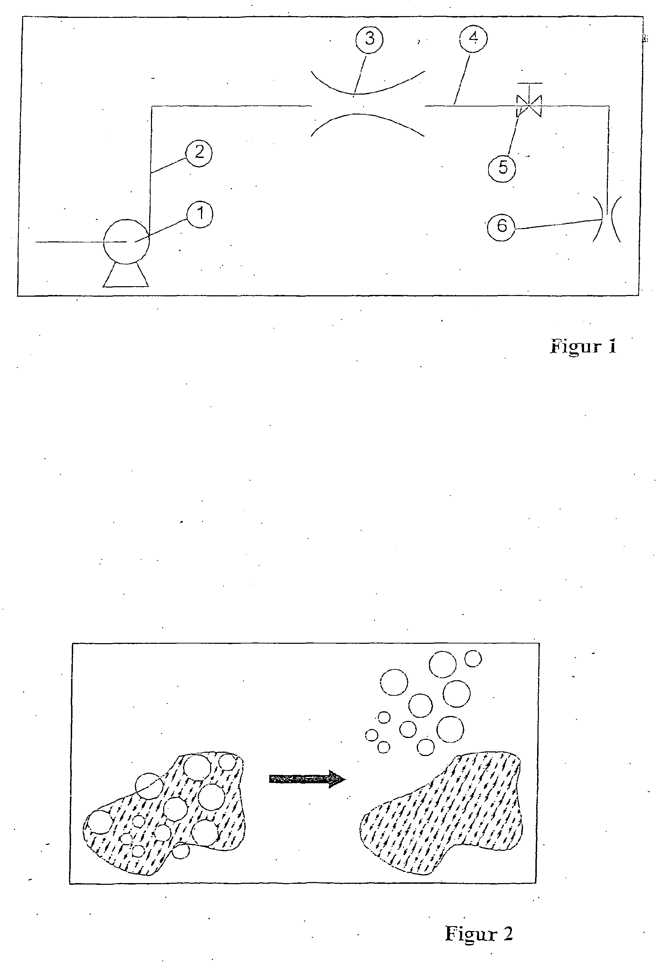

Figur 1 zeigt eine schematische Darstellung der erfindungsgemäßen Vorrichtung. Die Pumpe (1) fördert Abwasser, bevorzugt Belebtschlamm oder eine Mischbiozönose, bspw. aus einem Belebungsbecken oder einem Nachklärbecken (nicht gezeigt), über eine Rohrleitung (2) durch eine Düse (3) in eine Rohrleitung (4). In der Rohrleitung (4) kann eine Drossel (5) vorgesehen sein. Das behandelte Abwasser gelangt nachfolgend aus der Rohrleitung (4) über eine Abgabevorrichtung (6) vorzugsweise zurück in das Entnahmebecken, d.h. bspw. das Belebungsbecken bzw. das Nachklärbecken. Selbstverständlich kann das behandelte Abwasser über die Abgabevorrichtung (6) auch in ein weiteres Auffangbecken abgegeben werden.

Die Pumpe (1) kann sowohl vor der Düse (3) als auch nach der Düse (3) in der Rohrleitung(2) oder (4) angeordnet sein. Die Rohrleitung (2) weist einen Innendurchmesser auf, der bevorzugt größer als der Innendurchmesser der Rohrleitung (4) ist, wobei der Innendurchmesser der Rohrleitung (4) größer als der geringste Innendurchmesser der Düse (3) ist.

Als Pumpe (1) kann jede für die Verwendung in einem Klärbecken geeignete Pumpe verwendet werden. Die von der Düse (3) abströmseitig angeordnete Rohrleitung (4) bewirkt aufgrund des in bezug auf die anströmseitig zur Düse (3) angeordnete Rohrleitung (2) verringerten Innendurchmessers eine verlängerte Einwirkdauer der in der Düse (3) erzeugten Kavitation auf das Abwasser bzw. die Mischbiozönose. Die Länge der Rohrleitung (4) kann in Abhängigkeit von der gewünschten Einwirkdauer der Kavitation eingestellt werden. Bspw. kann die Länge der Rohrleitung (4) variabel einstellbar sein. Hierdurch kann eine gewünschte Verkürzung der Filamentlänge fädiger Bakterien als auch eine zuverlässige Freisetzung der in dem durch die fädigen Mikroorganismen erzeugten Fadengeflecht eingeschlossenen Gasblasen erreicht werden. Weiterhin wird durch den an die fädigen Mikroorganismen angelegten Streß deren Wachstum verlangsamt.

Die Einwirkdauer der Kavitation kann u.a. über die Länge der abströmseitig von der Düse (3) angeordneten Rohrleitung (4) gesteuert werden. Weiterhin kann die Entstehung von Kavitationsblasen auch durch den durch die Drosselklappe (5) einstellbaren Druck in der Rohrleitung (4) gesteuert werden. Die Abgabevorrichtung (6), die bevorzugt als Sprühdüse ausgebildet ist, bewirkt bei Abgabe der behandelten Mischbiozönose aus der erfindungsgemäßen Vorrichtung eine Entweichung der aus den Schlammaggregaten gelösten Gasblasen.

Vor der Düse (3) kann ein Konfusor angeordnet sein, der den Innendurchmesser der das Abwasser zuführenden Rohrleitung (2), d.h. der zur Düse anströmseitig angeordneten Rohrleitung (2), reduziert. Hierdurch wird der Übergang zwischen zuführender Rohrleitung (2) und Düse (3) verbessert.

Weiterhin ist es bevorzugt, zwischen Düse (3) und Drosselklappe (5) einen Diffusor anzuordnen, der den Innendurchmesser der von der Düse (3) abströmseitig angeordneten Rohrleitung (4) erweitert. Durch die Erweiterung des Innendurchmessers der Rohrleitung (4) wird der Druck in dem Abwasser, bevorzugt Belebtschlamm oder eine Mischbiozönose, erhöht und die Kavitation beendet. Dadurch kann ein Einwirken von Kavitation, d.h. ein Auftreffen von Kavitationsblasen auf die Drosselklappe (5) und mithin Schäden. bspw. Abrasion an der Drosselklappe (5) zuverlässig verhindert werden.

Bevorzugt wird der Innendurchmesser der Rohrleitung (4) nach dem Diffusor auf einen Innendurchmesser erweitert, der in etwa dem Innendurchmesser der anströmseitig zur Düse (3) angeordneten Rohrleitung (2) entspricht.

Über die Länge der Rohrleitung (4) mit reduziertem Innendurchmesser zwischen Düse (3) und Diffusor kann die Dauer der Kavitation und damit die Einwirkdauer der Kavitation gesteuert werden. Die Länge der Rohrleitung (4) zwischen Düse (3) und Diffusor kann veränderbar ausgestaltet sein, bspw. teleskopartig ausgebildet sein.

Grundsätzlich ist es auch möglich, mehrere Düsen (3), bspw. zwei oder drei Düsen, in einem Rohrleitungssystem hintereinander anzuordnen. Es hat sich jedoch gezeigt, daß eine Verringerung von aufgetretenem Blähschlamm, Schwimmschlamm und/oder Schaum bzw. eine Vermeidung des Auftretens von Blähschlamm, Schwimmschlamm und/oder Schaum durch die erfindungsgemäße Ausgestaltung bereits mit einer Düse (3) erreicht wird.

Die erfindungsgemäße Vorrichtung ist in konstruktiver Hinsicht überraschend einfach. Das erfindungsgemäße Verfahren bzw. die erfindungsgemäße Vorrichtung eignen sich mithin äußerst vorteilhaft für einen im wesentlichen wartungsfreien und mithin kostengünstigen Betrieb. Vorteilhaft wird für den Betrieb kein geschultes Personal benötigt. Insofern kann die vorliegende Erfindung vorteilhaft sowohl bei großen als auch bei kleinen Kläranlagen verwendet werden.

Figur 2 zeigt eine schematische Darstellung der Freisetzung von in Schlammaggregaten eingeschlossenen oder anhaftenden Gasblasen, die durch Beaufschlagung mit einem Scherfeld aus den Schlammaggregaten freigesetzt werden. Durch die Freisetzung der in den Schlammaggregaten eingeschlossenen oder anhaftenden Gasblasen wird der Auftrieb der Schlammaggregate wesentlich verringert.

Beim Durchströmen des Abwassers, bevorzugt des Belebtschlamms oder der Mischbiozönose, durch die Düse (3) kommt es weiterhin zu einer Druckabsenkung in dem durchtretenden Abwasser, was eine Ablösung der Gasblasen von den Schlammaggregaten weiter fördert. D.h., das Zusammenwirken der durch die Düse (3) in dem Abwasser induzierten Turbulenz mit der Verringerung des Drucks führt zu einer gewünschten Entgasung der Schlammaggregate bzw. Freisetzung der anhaftenden oder eingeschlossenen Gasblasen.

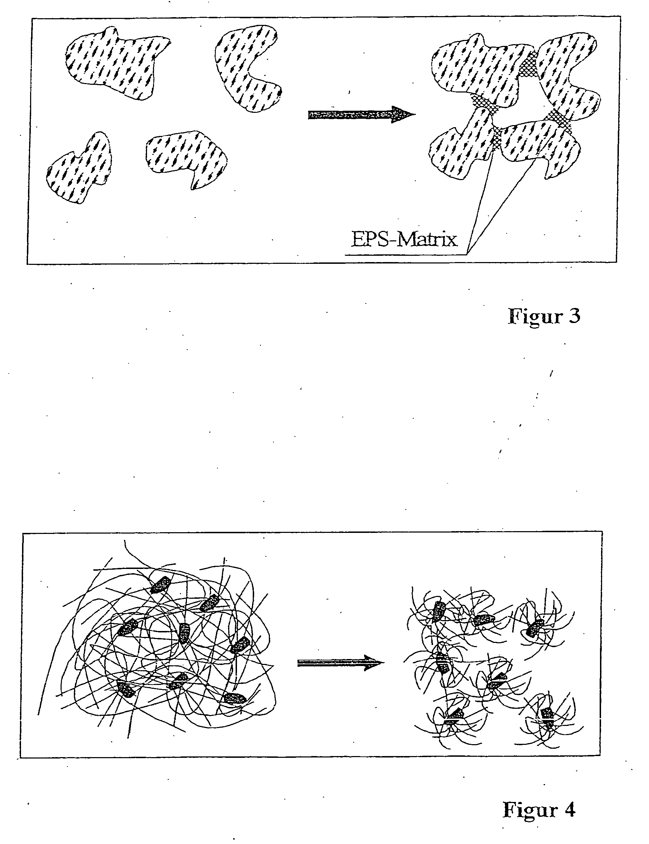

Figur 3 zeigt die durch die Sekretion von extrazellulären polymeren Substanzen (EPS) durch fädige Mikroorganismen bewirkte verbesserte Flockung von zerkleinerten Schlammaggregaten. Durch die in der Düse (3) induzierte Scherbeanspruchung des Abwassers, bevorzugt des Belebtschlamms oder der Mischbiozönose, werden die in dem Abwasser enthaltenen Mikroorganismen einem Streß unterworfen. Der Streß induziert bei Mikroorganismen die Sekretion von EPS. Die Sekretion von EPS führt zu einer flockenartigen Adhäsion von zerkleinerten Schlammaggregaten.

Diese Flockenbildung ist für die gewünschte Sedimentation des Klärschlamms sehr vorteilhaft. Die durch EPS bewirkte Zusammenlagerung von zerkleinerten Schlammaggregaten wirkt einer Bildung von zu kleinen Flocken entgegen, die aufgrund der zu geringen Größe nicht bzw. nicht innerhalb eines gewünschten Zeitraums sedimentieren.

Figur 4 zeigt schematisch die Verkürzung der Länge der Filamente von fädigen Mikroorganismen durch Anwendung des erfindungsgemäßen Verfahrens. Neben der Verkürzung der Filamentlänge wird auch das gebildete Fadengeflecht aufgebrochen.

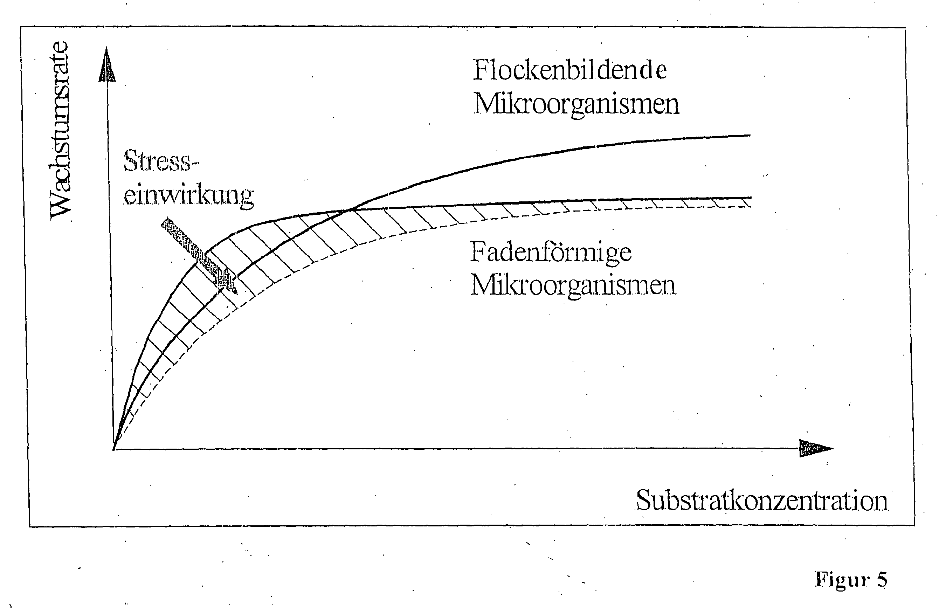

Figur 5 zeigt den Einfluß des erfindungsgemäßen Verfahrens auf die Wachstumskinetik von flockenbildenden Mikroorganismen und fädigen bzw. fadenförmigen Mikroorganismen. Figur 5 zeigt, daß die Wachstumsrate von flockenbildenden Mikroorganismen durch das erfindungsgemäße Verfahren nicht bzw. nicht wesentlich beeinflußt wird.

Hingegen hat das erfindungsgemäße Verfahren einen deutlichen Einfluß auf die Wachstumsrate von fädigen Mikroorganismen. Durch das in der erfindungsgemäßen Vorrichtung angelegte Scherfeld und die vorzugsweise auftretende Kavitation wird das Wachstum von fädigen Mikroorganismen deutlich gegenüber flockenbildenden Mikroorganismen zurückgedrängt. Dies führt zu einer gewünschten Veränderung der relativen Zusammensetzung der Mischbiozönose. Das relative Verhältnis von fädigen Mikroorganismen zu flockenbildenden Mikroorganismen wird erniedrigt.

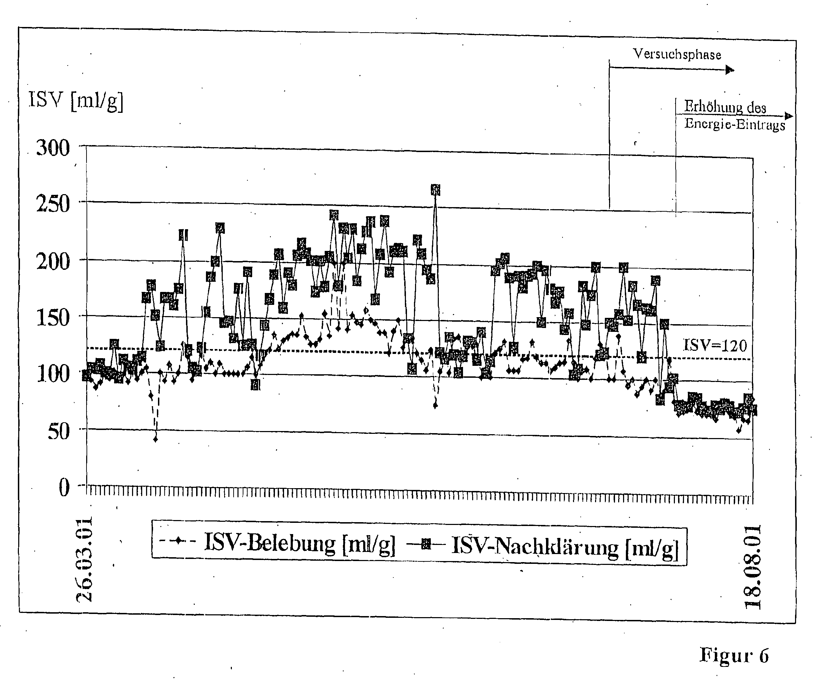

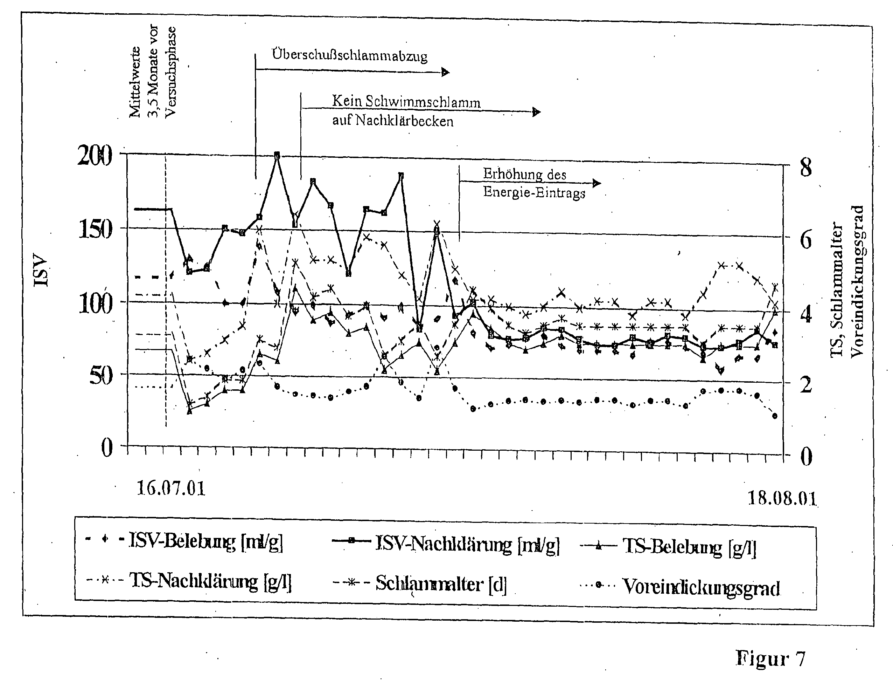

Figur 6 und Figur 7 zeigen Meßkurven, die in einer Versuchskläranlage gemessen wurden.

Das erfindungsgemäße Verfahren wurde an einer Kläranlage mit ca. 12.000 angeschlossenen Einwohnergleichwerten durchgeführt. Das Abwasser im Zulauf dieser Kläranlage war stark mit Abwässern aus der Textilindustrie belastet und führte seit mehr als 10 Jahren zu großen Blähschlamm- und Schwimmschlammproblemen.

Bei der Versuchskläranlage lag der Schlammvolumenindex (ISV) häufig über einem Wert von 120 ml/g. Die sich auf dem Nachklärbecken bildende Schlammdecke wurde bis zu dreimal täglich entfernt. Die Dicke der Schwimmschlammdecke konnte bis zu 5 cm und mehr anwachsen.

Die erfindungsgemäße Vorrichtung wurde direkt in dem Belebungsbecken der Kläranlage angeordnet. Innerhalb der ersten beiden Wochen wurde die erfindungsgemäße Vorrichtung täglich nur 4 Stunden betrieben (Leistung der Pumpe 3,75 kW bei einem Volumenstrom von 10,6 I/s). Der Innendurchmesser der vor der Düse angeordneten Rohrleitung betrug 100 mm, der Innendurchmesser der nach der Düse angeordneten Rohrleitung betrug 50 mm. Der engste Durchmesser der verwendeten Düse betrug 25 mm. Die Düse wies die Geometrie einer Lavaldüse auf.

Vor der Düse war ein Konfusor angeordnet, der den Innendurchmesser der zuführenden Rohrleitung von 100 mm auf 50 mm reduzierte. Abströmseitig von der Düse war in der Rohrleitung eine Drossel angeordnet. Zwischen Düse und Drossel war ein Diffusor angeordnet, der den Innendurchmesser der Rohrleitung von 50 mm wieder auf 100 mm erweiterte. Der Abstand zwischen Düse und Diffusor bzw. die Länge der Rohrleitung mit 50 mm Innendurchmesser betrug 100 cm.

Bereits nach 3 Tagen Versuchsdauer konnte eine deutlich verminderte Fadenlänge von langfädigen Strukturen im Belebtschlamm mikroskopisch beobachtet werden. Dieser Umstand machte sich in einem graduell abnehmenden Schlammvolumenindex (ISV) bemerkbar.

Nach 2 Versuchswochen wurde die erfindungsgemäße Vorrichtung auf Dauerbetrieb umgestellt und der Energieeintrag wurde auf etwa 350 kJ/m³ Mischbiozönose erhöht (Figur 6).

Der Schlammvolumenindex (ISV), der das Volumen des sedimentierten Schlamms von 1 I Inhalt nach 30 Min. Sedimentation, bezogen auf die Ausgangskonzentration beschreibt, ist nach Erhöhung des Energieeintrags auf unter deutlich 100 ml/g gesunken. Bei Werten über 120 ml/g kann es zu erheblichen Problemen der Absetzeigenschaften des Blähschlamms im Nachklärbecken kommen.

Bereits am darauffolgenden Tag konnte eine drastische Längenreduzierung der Filamentlänge von fädigen Mikroorganismen mikroskopisch beobachtet werden. Der Schlammvolumenindex hatte einen Wert von ca. 80 ml/g und hielt diesen Wert über die kommenden Wochen hinweg aufrecht. Dieser Wert liegt deutlich unter dem Schwellenwert von 120 ml/g, ab dem ein Blähschlammverhalten zu erwarten ist. Sowohl im Belebungsbecken als auch im Nachklärbecken kam es zu einer signifikanten Reduktion des Schlammvolumenindexes auf etwa 80 ml/g.

Es hat sich völlig überraschend gezeigt, daß der Energieeintrag in die Mischbiozönose deutlich unter 1.000 kJ/m³ liegen kann, um das Auftreten von Bläh- und Schwimmschlamm zu vermeiden. In Abhängigkeit von dem Anteil an fädigen Mikroorganismen in der Mischbiozönose kann der Energieeintrag beispielsweise in einem Bereich von etwa 200 kJ/m³ Mischbiozönose bis etwa 800 kJ/m³ Mischbiozönose, bevorzugt von etwa 250 kJ/m³ Mischbiozönose bis 600 kJ/m³ Mischbiozönose, weiter bevorzugt von etwa 300 kJ/m³ bis etwa 500 kJ/m³ Mischbiozönose, liegen. Die Geschwindigkeit des durch die Rohrleitung (4) geförderten Abwassers bzw. der Mischbiozönose liegt dabei in einem Bereich von etwa 3 m/s bis 10 m/s, bevorzugt von etwa 4 m/s bis 8 m/s, weiter bevorzugt von etwa 5 m/s bis 7 m/s.

Aus Figur 7 geht hervor, daß es auch bei weiteren Prozeßparametern zu einer Stabilisierung kam, d.h. das erfindungsgemäße Verfahren zu einem stabilen Betrieb der Versuchsanlage führte.

Zu sehen ist deutlich, daß aufgrund der verbesserten Absetzeigenschaften Überschußschlamm abgezogen werden konnte und es damit zu einer Stabilisierung sämtlicher Prozeßparameter kam. Bei Erhöhung des Energieeintrags auf etwa 350 kJ/m³ durch Erhöhung der Pumpleistung erfolgte eine Glättung der erhaltenen Meßkurven, d.h. beim Schlammindex im Belebungsbecken (ISV-Belebung), Schlammindex im Nachklärbecken (ISV-Nachklärung), Trockensubstanz im Belebungsbecken (TS-Belebung), Trockensubstanz im Nachklärbecken (TS-Nachklärung), beim Schlammalter und beim Voreindickungsgrad. Die Mischbiozönose ist deutlich gesundet.

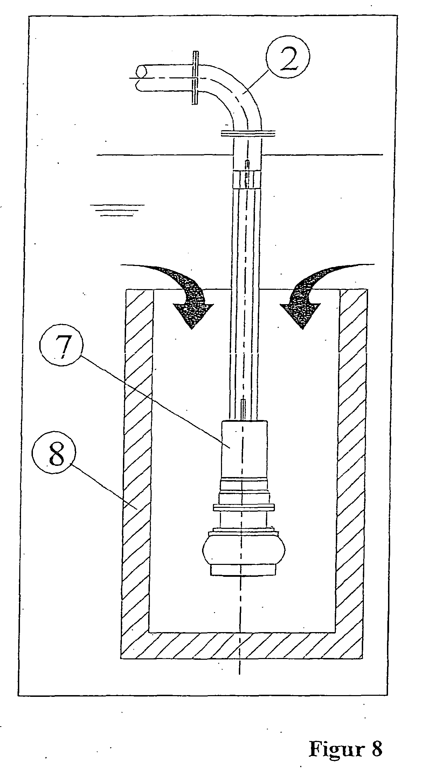

Figur 8 zeigt eine in einem unter der Oberfläche der Mischbiozönose angeordneten Schacht (8) angeordnete Pumpe (7). Der Schacht (8) wird im Sinne der Erfindung als getauchter Schacht (8) bezeichnet. In dem getauchten Schacht (8) ist eine Pumpe (7) angeordnet, die das Abwasser, bevorzugt den Belebtschlamm oder die Mischbiozönose, in die Rohrleitung (2) fördert. Zwischen Pumpe (7) und den Innenwandungen des getauchten Schachts (8) bildet sich eine Entgasungszone aus. In der Entgasungszone werden zumindest teilweise in dem Abwasser bzw. der Mischbiozönose mitgeführte oder enthaltene Gasblasen abgetrennt und freigesetzt, die durch den in Richtung zur Oberfläche offenen Schacht entweichen.

## Patentansprüche

1. Verfahren zur Behandlung von fädige und flockenbildende Mikroorganismen-haltigem Abwasser, Klärschlamm oder in Biogasanlagen verwendeten organischen Substraten,
**dadurch gekennzeichnet,**
**daß** das Abwasser, der Klärschlamm oder die Substrate in einem Strömungskanal (2, 4) geführt und in dem Strömungskanal (2, 4) einer Scherbeanspruchung, die ausreichend ist, um die fädigen Mikroorganismen teilweise zu zerstören und um die Filamentlänge der fädigen Mikroorganismen zu verkürzen, unterworfen wird bzw. werden, wobei
die Scherbeanspruchung durch eine turbulente Strömung in dem Strömungskanal (2, 4) erzeugt wird und
das Abwasser, der Klärschlamm oder die Substrate durch wenigstens eine in dem Strömungskanal (2,4) angeordnete Verengung (3) geführt wird bzw. werden, wobei
die Fließgeschwindigkeit des Abwassers, des Klärschlamms oder der organischen Substrate bei Durchtritt durch die Verengung (3) des Strömungskanals (2, 4) unter abnehmendem Druck erhöht wird, wobei nachfolgend in dem Abwasser, in dem Klärschlamm oder in den organischen Substraten Kavitation erzeugt wird,
wobei
die Entstehung der Kavitation einstellbar ist, indem der Innendurchmesser des Strömungskanals (2, 4) so gewählt wird, daß der Innendurchmesser an der Verengung (3) am geringsten ist und der Innendurchmesser des Strömungskanals (2) anströmseitig vor der Verengung (3) des Innendurchmessers größer ist als der Innendurchmesser des Strömungskanals (4) abströmseitig nach der Verengung (3).

2. Verfahren gemäß Anspruch 1,
**dadurch gekennzeichnet,**
**daß** die Dauer der Kavitation über die Länge des abströmseitig nach der Verengung (3) angeordneten Strömungskanals (4) einstellbar ist.

3. Verfahren gemäß Anspruch 2,
**dadurch gekennzeichnet,**
**daß** der Innendurchmesser der Verengung (3) im Strömungskanal (2, 4) variierbar ist.

4. Verfahren gemäß einem der vorhergehenden Ansprüche,
**dadurch gekennzeichnet,**
**daß** in dem Abwasser, Klärschlamm oder in den organischen Substraten mitgeführte oder enthaltene Gasblasen vor dem Einbringen in den Strömungskanal (2, 4) wenigstens teilweise entfernt werden.

5. Verfahren gemäß Anspruch 4,
**dadurch gekennzeichnet,**
**daß** das Abwasser, der Klärschlamm oder die organischen Substrate durch Fördern aus einem getauchten Schacht (8) wenigstens teilweise von mitgeführten oder enthaltenen Gasblasen befreit wird bzw. werden.

6. Verfahren gemäß einem der vorhergehenden Ansprüche,
**dadurch gekennzeichnet,**
**daß** die Reynoldszahl des durch den Strömungskanal (2, 4) geführten Abwassers, Klärschlamms oder der organischen Substrate wenigstens 100.000, bevorzugt wenigstens 250.000, weiter bevorzugt wenigstens 500.000, beträgt.

7. Verfahren gemäß einem der vorhergehenden Ansprüche,
**dadurch gekennzeichnet,**
**daß** das Abwasser, der Klärschlamm oder die organischen Substrate nach Durchleitung durch den Strömungskanal (2,4) versprüht wird bzw. werden.

8. Verfahren gemäß einem der vorhergehenden Ansprüche,
**dadurch gekennzeichnet,**
**daß** die wenigstens eine Verengung (3) eine Düse ist.

9. Verfahren gemäß einem der vorhergehenden Ansprüche,
**dadurch gekennzeichnet,**
**daß** der Strömungskanal (2,4) eine Rohrleitung ist.

10. Verfahren gemäß einem der vorhergehenden Ansprüche,
**dadurch gekennzeichnet,**
**daß** das Abwasser Belebtschlamm oder eine Mischbiozönose ist.

11. Verfahren gemäß einem der vorhergehenden Ansprüche,
**dadurch gekennzeichnet,**
**daß** die fädigen Mikroorganismen durch Aufbrechen der Zellmembranen teilweise zerstört werden.

12. Verfahren gemäß einem der vorhergehenden Ansprüche,
**dadurch gekennzeichnet,**
**daß** die von fädigen Mikroorganismen gebildeten Fadengeflechte aufgebrochen bzw. zerkleinert werden.

13. Verfahren gemäß einem der vorhergehenden Ansprüche,
**dadurch gekennzeichnet,**
**daß** der Energieeintrag 500 kJ/m³ bis 800 kJ/m³ beträgt.

14. Vorrichtung zur Behandlung von fädige und flockenbildende Mikroorganismen-haltigem Abwasser, Klärschlamm oder in Biogasanlagen verwendeten organischen Substraten zur teilweisen Zerstörung der fädigen Mikroorganismen und zur Verkürzung der Filamentlänge der fädigen Mikroorganismen,
**dadurch gekennzeichnet,**
**daß** die Vorrichtung einen wenigstens eine Verengung (3) aufweisenden Strömungskanal (2,4) und eine Einrichtung (7) zum Fördern von Abwasser, Klärschlamm oder organischen Substraten durch den Strömungskanal (2,4) aufweist, wobei
der Innendurchmesser des Strömungskanals (2,4) an der Verengung (3) am geringsten ist und der Innendurchmesser des Strömungskanals (2) anströmseitig vor der Verengung (3) größer ist als der Innendurchmesser des Strömungskanals (4) abströmseitig nach der Verengung (3) und wobei die wenigstens eine Verengung (3) einen Abschnitt mit konvergierendem und divergierendem Innendurchmesser umfaßt, wobei abströmseitig der Abschnitt mit konvergierendem Innendurchmesser und abströmseitig der Abschnitt mit divergierendem Innendurchmesser angeordnet ist.

15. Vorrichtung gemäß Anspruch 14,
**dadurch gekennzeichnet,**
**daß** das Verhältnis des Innendurchmessers des Strömungskanals (2) vor der Verengung (3) und des Innendurchmessers des Strömungskanals (4) nach der Verengung (3) etwa 5:1 bis etwa 1,2:1 beträgt.

16. Vorrichtung gemäß Anspruch 15,
**dadurch gekennzeichnet,**
**daß** das Verhältnis etwa 3:1 bis etwa 1,5:1, bevorzugt 2:1, beträgt

17. Vorrichtung gemäß einem der Ansprüche 14 bis 16,
**dadurch gekennzeichnet,**
**daß** die Länge des Abschnitts mit konvergierendem Innendurchmesser kürzer ist als die Länge des Abschnitts mit divergierendem Innendurchmesser.

18. Vorrichtung gemäß einem der Ansprüche 14 bis 17,
**dadurch gekennzeichnet,**
**daß** abströmseitig von der Verengung (3) in dem Strömungskanal (4) eine Drossel (5) angeordnet ist.

19. Vorrichtung gemäß einem der Ansprüche 14 bis 18,
**dadurch gekennzeichnet,**
**daß** die Länge des abströmseitig nach der Verengung (3) angeordneten Strömungskanals (4) variabel einstellbar ist.

20. Vorrichtung gemäß einem der Ansprüche 14 bis 19,
**dadurch gekennzeichnet,**
**daß** die Verengung (3) eine Düse ist.

21. Vorrichtung gemäß Anspruch 20,
**dadurch gekennzeichnet,**
**daß** anströmseitig vor der Düse (3) ein Konfusor angeordnet ist.

22. Vorrichtung gemäß einem der Ansprüche 18 bis 21,
**dadurch gekennzeichnet,**
**daß** nach dem abströmseitig von der Verengung angeordneten Strömungskanal (4), bevorzugt vor der Drossel (5), ein Diffusor angeordnet ist.

23. Vorrichtung gemäß einem der Ansprüche 14 bis 22,
**dadurch gekennzeichnet,**
**daß** der Strömungskanal (2,4) eine Rohrleitung ist.

24. Vorrichtung gemäß einem der Ansprüche 14 bis 23,
**dadurch gekennzeichnet,**
**daß** die Einrichtung (7) zum Fördern des Abwassers, Klärschlamms oder der organischen Substrate eine Pumpe ist.

25. Vorrichtung gemäß Anspruch 24,
**dadurch gekennzeichnet,**
**daß** die Pumpe in einem getauchten Schacht (8) angeordnet ist.

## Claims

1. Method for the treatment of such effluent, sewage sludge or organic substrates used in biogas plants as contain filamentary and flocculating microorganisms,
**characterized**
**in that** the effluent, the sewage sludge or the substrates is/are led in a flow channel (2, 4) and is/are subjected in the flow channel (2, 4) to a shearing stress which is sufficient to partially destroy the filamentary microorganisms and to shorten the filament length of the filamentary microorganisms, the shearing stress being generated by a turbulent flow in the flow channel (2, 4) and the effluent, the sewage sludge or the substrates being led through at least one contraction (3) arranged in the flow channel (2, 4), the flow velocity of the effluent, the sewage sludge or the organic substrates being increased, under decreasing pressure, during passage through the contraction (3) of the flow channel (2, 4), cavitation subsequently being generated in the effluent, in the sewage sludge or in the organic substrates, it being possible for the occurrence of the cavitation to be set in that the inside diameter for the flow channel (2, 4) is selected such that the inside diameter is the smallest at the contraction (3) and the inside diameter of the flow channel (2) on the inflow side, upstream of the contraction (3) of the inside diameter is larger than the inside diameter of the flow channel (4) on the outflow side, downstream of the contraction (3).

2. Method according to Claim 1,
**characterized**
**in that** the duration of the cavitation can be set via the length of the flow channel (4) arranged on the outflow side, downstream of the contraction (3).

3. Method according to Claim 2, **characterized in that** the inside diameter of the contraction (3) in the flow channel (2, 4) can be varied.

4. Method according to one of the preceding claims,
**characterized**
**in that** gas bubbles entrained or contained in the effluent, in the sewage sludge or in the organic substrates are at least partially removed before introduction into the flow channel (2, 4).

5. Method according to Claim 4,
**characterized**
**in that** the effluent, the sewage sludge or the organic substrates is/are at least partially freed of entrained or contained gas bubbles by conveyance out of a submerged well (8).

6. Method according to one of the preceding claims,
**characterized**
**in that** the Reynold's number of the effluent, sewage sludge or organic substrates led through the flow channel (2, 4) is at least 100 000, preferably at least 250 000,- more preferably at least 500 000.

7. Method according to one of the preceding claims,
**characterized**
**in that** the effluent, the sewage sludge or the organic substrates is/are sprayed after passage through the flow channel (2, 4).

8. Method according to one of the preceding claims,
**characterized**
**in that** the at least one contraction (3) is a nozzle.

9. Method according to one of the preceding claims, **characterized in that** the flow channel (2, 4) is a pipeline.

10. Method according to one of the preceding claims,
**characterized**
**in that** the effluent is activated sludge or a mixed biozenosis.

11. Method according to one of the preceding claims, **characterized**
**in that** the filamentary microorganisms are partially destroyed by the break-up of the cell membranes.

12. Method according to one of the preceding claims, **characterized**
**in that** the filamentary intertwinings formed by filamentary microorganisms are broken up or comminuted.

13. Method according to one of the preceding claims, **characterized**
**in that** the amount of energy introduced is 500 kJ/m³ to 800 kJ/m³.

14. Device for the treatment of such effluent, sewage sludge or organic substrates used in biogas plants as contain filamentary and flocculating microorganisms for the partial destruction of the filamentary microorganisms and for shortening the filament length of the filamentary microorganisms,
**characterized**
**in that** the device has a flow channel (2, 4) having at least one contraction (3) and a means (7) for the conveyance of effluent, sewage sludge or organic substrates through the flow channel (2, 4), the inside diameter of the flow channel (2, 4) being the smallest at the contraction (3), and the inside diameter of the flow channel (2) on the inflow side, upstream of the contraction (3), being larger than the inside diameter of the flow channel (4) on the outflow side, downstream of the contraction (3) and the at least one contraction (3) comprises a segment with a converging and a diverging inside diameter, the segment with a converging inside diameter being arranged on the inflow side and the segment with a diverging inside diameter being arranged on the outflow side.

15. Device according to Claim 14,
**characterized**
**in that** the ratio of the inside diameter of the flow channel (2) upstream of the contraction (3) to the inside diameter of the flow channel (4) downstream of the contraction (3) is about 5:1 to about 1.2:1.

16. Device according to Claim 15,
**characterized**
**in that** the ratio is about 3:1 to about 1.5:1, preferably 2:1.

17. Device according to one of Claims 14 to 16, **characterized**
**in that** the length of the segment with a converging inside diameter is shorter than the length of the segment with a diverging inside diameter.

18. Device according to one of Claims 14 to 17, **characterized**
**in that** a throttle (5) is arranged, downstream of the contraction (3), in the flow channel (4).

19. Device according to one of Claims 14 to 18,
**characterized**
**in that** the length of the flow channel (4) arranged on the outflow side, downstream of the contraction (3), can be set variably.

20. Device according to one of Claims 14 to 19,
**characterized**
**in that** the contraction (3) is a nozzle.

21. Device according to Claim 20,
**characterized**
**in that** a confuser is arranged on the inflow side, upstream of the nozzle (3).

22. Device according to one of Claims 18 to 21,
**characterized**
**in that** a diffuser is arranged downstream of the flow channel (4) arranged on the outflow side of the contraction, preferably upstream of the throttle (5).

23. Device according to one of Claims 14 to 22, **characterized**
**in that** the flow channel (2, 4) is a pipeline.

24. Device according to one of Claims 14 to 23,
**characterized**
**in that** the means (7) for conveying the effluent, sewage or the organic substrates is a pump.

25. Device according to Claim 24,
**characterized**
**in that** the pump is arranged in a submerged well (8).

## Revendications

1. Procédé de traitement d'eaux usées contenant des micro-organismes filamenteux et floculants, de boues résiduaires ou de substrats organiques utilisés dans des installations biogaz,
**caractérisé en ce que**
les eaux usées, les boues résiduaires ou les substrats sont guidés dans un canal d'écoulement (2, 4) et soumis dans ce dernier (2, 4) à une sollicitation au cisaillement suffisante pour détruire partiellement les microorganismes filamenteux et réduire la longueur des filaments des microorganismes filamenteux,
la sollicitation au cisaillement étant générée par un courant turbulent dans le canal d'écoulement (2, 4) et
les eaux usées, les boues résiduaires ou les substrats étant guidés à travers au moins un rétrécissement (3) disposé dans le canal d'écoulement (2, 4),
la vitesse d'écoulement des eaux usées, des boues résiduaires ou des substrats organiques étant augmentée, à une pression en diminution, lors du passage à travers le rétrécissement (3) du canal d'écoulement (2, 4), une cavitation étant ensuite générée dans les eaux usées, les boues résiduaires ou les substrats organiques,
la formation de la cavitation étant réglable en choisissant le diamètre intérieur du canal d'écoulement (2, 4) de telle manière que le diamètre intérieur soit le plus faible au niveau du rétrécissement (3) et que le diamètre intérieur du canal d'écoulement (2) soit plus grand côté arrivée avant le rétrécissement (3) que le diamètre intérieur du canal d'écoulement (4) côté sortie après le rétrécissement (3).

2. Procédé selon la revendication 1,
**caractérisé en ce que**
la durée de la cavitation est réglable sur la longueur du canal d'écoulement (4) disposé côté sortie après le rétrécissement (3).

3. Procédé selon la revendication 2,
**caractérisé en ce que**
le diamètre intérieur du rétrécissement (3) dans le canal d'écoulement (2, 4) peut varier.

4. Procédé selon l'une quelconque des revendications précédentes,
**caractérisé en ce que**
des bulles de gaz entraînées ou contenues dans les eaux usées, les boues résiduelles ou les substrats organiques sont enlevées au moins partiellement avant l'introduction dans le canal d'écoulement (2, 4).

5. Procédé selon la revendication 4,
**caractérisé en ce que**
les eaux usées, les boues résiduaires ou les substrats organiques sont libérés au moins partiellement de bulles de gaz entraînées ou contenues dans ces derniers par évacuation d'une cuve (8) immergée.

6. Procédé selon l'une quelconque des revendications précédentes,
**caractérisé en ce que**
le nombre de Reynolds des eaux usées, des boues résiduaires ou des substrats organiques guidés à travers le canal d'écoulement (2, 4) est d'au moins 100.000, de préférence d'au moins 250.000, mieux encore d'au moins 500.000.

7. Procédé selon l'une quelconque des revendications précédentes,
**caractérisé en ce que**
les eaux usées, les boues résiduaires ou les substrats organiques sont vaporisés après avoir traversé le canal d'écoulement (2, 4).

8. Procédé selon l'une quelconque des revendications précédentes,
**caractérisé en ce que**
le rétrécissement (3) minimum est une tuyère.

9. Procédé selon l'une quelconque des revendications précédentes,
**caractérisé en ce que**
le canal d'écoulement (2, 4) est une conduite.

10. Procédé selon l'une quelconque des revendications précédentes,
**caractérisé en ce que**
les eaux usées sont des boues activées ou une biocénose mixte.

11. Procédé selon l'une quelconque des revendications précédentes,
**caractérisé en ce que**
les microorganismes filamenteux sont partiellement détruits par rupture des membranes cellulaires.

12. Procédé selon l'une quelconque des revendications précédentes,
**caractérisé en ce que**
les entrelacements formés de microorganismes filamenteux sont rompus et/ou fractionnés.

13. Procédé selon l'une quelconque des revendications précédentes,
**caractérisé en ce que**
l'apport en énergie est de 500 kJ/m³ à 800 kJ/m³.

14. Dispositif de traitement d'eaux usées contenant des micro-organismes filamenteux et floculants, de boues résiduaires ou de substrats organiques utilisés dans des installations biogaz pour détruire partiellement les microorganismes filamenteux et réduire la longueur des filaments des microorganismes filamenteux,
**caractérisé en ce que**
le dispositif présente un canal d'écoulement (2, 4) présentant au moins un rétrécissement (3) et un dispositif (7) de transport d'eaux usées, de boues résiduaires ou de substances organiques à travers le canal d'écoulement (2, 4),
le diamètre intérieur du canal d'écoulement (2,4) étant le plus petit au niveau du rétrécissement (3) et le diamètre intérieur du canal d'écoulement (2) côté arrivée devant le rétrécissement (3) étant plus grand que le diamètre intérieur du canal d'écoulement (4) côté sortie après le rétrécissement (3) et le rétrécissement (3) minimum comportant un tronçon avec un diamètre intérieur convergent et divergent, le tronçon avec un diamètre intérieur convergent étant disposé côté entrée et le tronçon avec un diamètre intérieur divergent côté sortie.

15. Dispositif selon la revendication 14,
**caractérisé en ce que**
le rapport entre le diamètre intérieur du canal d'écoulement (2) avant le rétrécissement (3) et le diamètre intérieur du canal d'écoulement (4) après le rétrécissement (3) est d'environ 5:1 à environ 1,2:1.

16. Dispositif selon la revendication 15,
**caractérisé ce que**
le rapport est d'environ 3:1 à environ 1,5:1, de préférence de 2:1.

17. Dispositif selon l'une quelconque des revendications 14 à 16,
**caractérisé en ce que**
la longueur du tronçon avec un diamètre intérieur convergent est plus petite que la longueur du tronçon avec un diamètre intérieur divergent.

18. Dispositif selon l'une quelconque des revendications 14 à 17,
**caractérisé en ce**
**qu'**un étrangleur (5) est disposé côté sortie du rétrécissement (3) dans le canal d'écoulement (4).

19. Dispositif selon l'une quelconque des revendications 14 à 18,
**caractérisé en ce que**
la longueur du canal d'écoulement (4) disposé côté sortie après le rétrécissement (3) est réglable de manière variable.

20. Dispositif selon l'une quelconque des revendications 14 à 19,
**caractérisé en ce que**
le rétrécissement (3) est une tuyère.

21. Dispositif selon la revendication 20,
**caractérisé en ce**
**qu'**un confuseur est disposé côté arrivée devant la tuyère (3).

22. Dispositif selon l'une quelconque des revendications 18 à 21,
**caractérisé en ce**
**qu'**un diffuseur est disposé après le canal d'écoulement (4) disposé côté sortie du rétrécissement, de préférence devant l'étrangleur (5).

23. Dispositif selon l'une quelconque des revendications 14 à 22,
**caractérisé en ce que**
le canal d'écoulement (2, 4) est une conduite.

24. Dispositif selon l'une quelconque des revendications 14 à 23,
**caractérisé en ce que**
le dispositif (7) de transport des eaux usées, des boues résiduaires ou des substrats organiques est une pompe.

25. Dispositif selon la revendication 24,
**caractérisé en ce que**
la pompe est disposée dans une cuve (8) immergée.
